# EUROPÄISCHE PATENTSCHRIFT

(11) **EP 0 690 704 B1**
(45) Veröffentlichungstag und Bekanntmachung des Hinweises auf die Patenterteilung: **09.09.1998**
(21) Anmeldenummer: 94908313.3
(22) Anmeldetag: 16.02.1994
(51) Int. Cl.: A61F 5/01, A61F 2/64

(54) **ORTHESE ODER EXOPROTHESE FÜR DAS MENSCHLICHE KNIEGELENK**
ORTHESIS OR EXOPROSTHESIS FOR THE HUMAN KNEE JOINT
ORTHESE OU EXOPROTHESE DE L'ARTICULATION DU GENOU HUMAIN

(30) Priorität: 24.03.1993 DE 4309577
(43) Veröffentlichungstag der Anmeldung: 10.01.1996
(73) Patentinhaber: Theusner, Joachim, Dr., 80539 München (DE); Kubein-Meesenburg, Dietmar, Prof. Dr., 37547 Kreiensen (DE); Nägerl, Hans, Dr., 37130 Gleichen (DE)
(72) Erfinder: KUBEIN-MEESENBURG, Dietmar, D-37547 Kreiensen (DE); NÄGERL, Hans, D-37130 Gleichen/OT (DE)
(74) Vertreter: Braun, Dieter, Dipl.-Ing.
(86) Internationale Anmeldenummer: EP9400433
(87) Internationale Veröffentlichungsnummer: WO9421200

(56) Entgegenhaltungen:
- EP-A- 0 482 809
- DE-A- 2 317 561
- DE-C- 412 362
- FR-A- 2 231 356
- US-A- 5 168 865

## Beschreibung

Die vorliegende Erfindung betrifft eine Exoprothese für das menschliche Kniegelenk gemäß dem Oberbegriff des Anspruchs 1.

Eine derartige Exoprothese ist aus der US-A-5 168 865 bekannt. Bei dieser Exoprothese sind zur Einschränkung des Bewegungsraums Anschläge für das Koppelglied vorgesehen und, da das bekannte Gelenk nur in etwa 10 bis 20% reine Gelenkfunktion als Kniegelenk arbeitet und im übrigen Bereich ein Scharniergelenk darstellt, das nicht selbstregulierend ist, sondern Bewegungsanschläge benötigt.

Die weiterhin bekannten Exoprothesen für das menschliche Kniegelenk sind Mehrgelenkanordnungen unter Verwendung von Kugelgelenken und/oder Scharniergelenken. Derartige Gelenkanordnungen sind ebenfalls nicht geeignet, die tatsächliche Gelenkfunktion des menschlichen Kniegelenks im wesentlichen naturgetreu nachzubilden, so daß diese Exoprothesen eine erhebliche Gehbehinderung verursachen.

Der Erfindung liegt die Aufgabe zugrunde, eine Exoprothese für das menschliche Kniegelenk zu schaffen, die eine Gelenkfunktion besitzt, die mit der natürlichen Gelenkfunktion des menschlichen Knies im wesentlichen übereinstimmt.

Erfindungsgemäß wird dies dadurch erreicht, daß in der gestreckten Lage des Kniegelenks die Gelenkachsen parallel zueinander in zwei parallelen Ebenen, die jeweils die Achsen des Medial- bzw. des Lateralgelenks enthalten, verlaufen, und das Lateralgelenk gegenüber dem Medialgelenk nach posterior (d.h. nach hinten) um ein Maß Δy, das dem Abstand der genannten parallelen Ebenen entspricht, sowie das Medialgelenk gegenüber dem Lateralgelenk hinsichtlich seiner Gelenkachsen in Richtung auf den Oberschenkel um ein Maß Δx, das dem Anstand der Gelenkachsen voneinander entspricht, versetzt ist. Das mediale Gelenk ist aufgrund seiner Gelenkgeometrie druckstabil, wohingegen das laterale Gelenk labil ist. Durch die erfindungsgemäße Ausgestaltung wird sichergestellt, daß die Exoprothese nur eine Bewegungsfreiheit in einer Gelenkebene besitzt und gleichzeitig eine hohe mechanische Stabilität erreicht wird, wobei eine große Variationsbreite zur Anpassung an individuelle Gegebenheiten vorhanden ist.

Vorteilhafte Ausführungen der Erfindung sind in den Unteransprüchen enthalten.

Anhand der in den beiliegenden Zeichnungen dargestellten Ausführungsbeispiele wird die Erfindung näher erläutert. Es zeigen:
- Fig. 1: eine Frontalansicht einer erfindungsgemäßen Exoprothese im am menschlichen Bein befestigten Zustand, zum Teil geschnitten,
- Fig. 2: eine Seitenansicht gemäß des Pfeiles II in Fig. 1,
- Fig. 3: eine Seitenansicht einer weiteren Ausführungsform einer erfindungsgemäßen Prothese im am menschlichen Bein befestigten Zustand,
- Fig. 4: einen Schnitt durch eine weitere Ausführungsform einer erfindungsgemäßen Exoprothese als Ersatz des menschlichen Kniegelenks,
- Fig. 5: eine Seitenansicht auf die erfindungsgemäße Exoprothese gemäß Fig. 4 in Richtung des Pfeiles V.
- Fig. 6: eine Prinzipdarstellung der Gelenkgeometrie in Seitenansicht gemäß Fig. 2

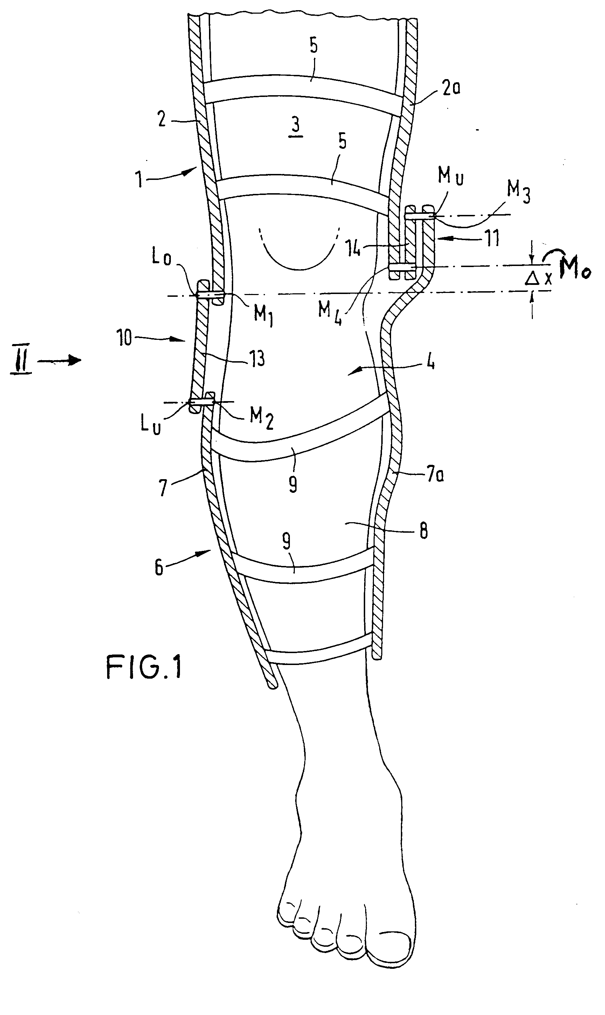

Wie sich aus Fig. 1 ergibt, besteht eine erfindungsgemäße Exoprothese aus einem Oberschenkelteil 1, das im dargestellten Ausführungsbeispiel aus zwei Oberschenkelschienen 2, 2a besteht, die jeweils an der medialen (inneren) und lateralen (äußeren) Seite eines Oberschenkels 3 eines menschlichen Beins 4 verlaufen und durch Querschienen 5 miteinander verbunden sind, die den Oberschenkel 3 umfassen. Das Unterschenkelteil 6 ist aus zwei Unterschenkelschienen 7, 7a gebildet, und zwar an der medialen und lateralen Seite eines Unterschenkels 8 des Beins 4, die durch Querschienen 9 verbunden sind, wobei die Querschienen 9 wiederum den Unterschenkel 8 umfassen. Die laterale Ober- und Unterschenkelschiene 2, 7 sowie die mediale Oberschenkel- und Unterschenkelschiene 2a, 7a sind jeweils durch ein Zwei-Gelenk, und zwar das Lateralgelenk 10 und das Medialgelenk 11 verbunden, die in zwei parallelen Ebenen nebeneinander liegen, d. h. die beiden parallel angeordneten Zwei-Gelenke bilden eine Vier-Gelenkanordnung. Das Lateralgelenk 10 und das Medialgelenk 11 besitzen die Gelenkgeometrie einer Gelenkkette mit zwei Gelenkachsen, eine sogenannte dimere Gelenkkette. Hierbei ist das Lateralgelenk 10 als gestreckte, dimere Gelenkkette mit den beiden Gelenkachsen Lₒ, Lᵤ ausgebildet, wobei die Gelenkachse Lₒ mit der Gelenkachse Lᵤ durch ein Koppelglied 13 gelenkig verbunden ist. Die Gelenkachsen Lₒ und Lᵤ besitzen die Rotationszentren M₁ bzw. M₂ und verlaufen senkrecht zur sagitalen Ebene bzw. senkrecht zur Schwenkebene des Beines 6. In der dargestellten, gestreckten Beinstellung befinden sich die Gelenkachsen Lₒ und Lᵤ im Abstand voneinander, und zwar Lₒ in Richtung auf den Oberschenkel 3 versetzt und die Gelenkachse Lᵤ in Richtung auf den Unterschenkel versetzt. Das Koppelglied 13 ist in der Standstellung vertikal angeordnet.

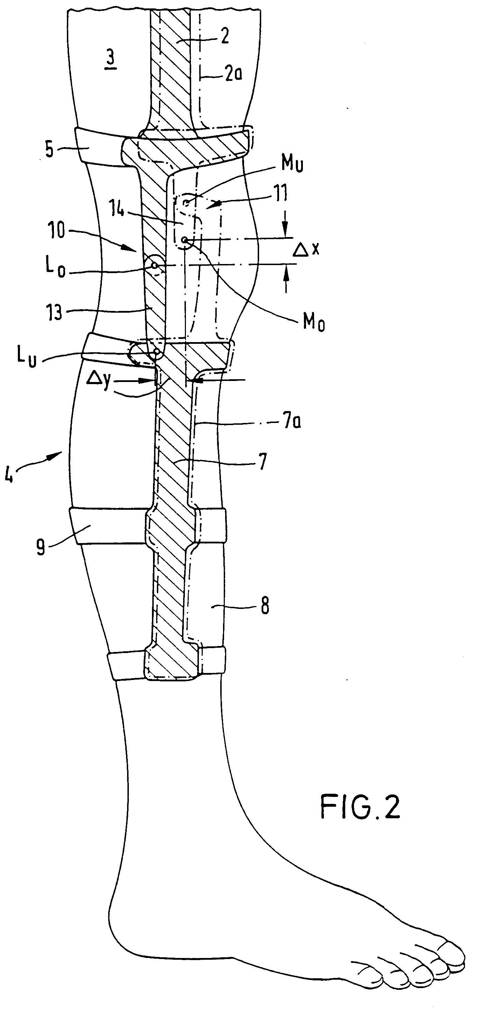

Das Medialgelenk 11 ist als überschlagene, dimere Gelenkkette ausgebildet, wobei die Gelenkachse Mᵤ der Unterschenkelschiene 7a in Richtung auf den Oberschenkel 3 versetzt gegenüber der Gelenkachse Mₒ der Oberschenkelschiene 2a ist, und zwar unter Bezug auf die dargestellte gesteckte Beinstellung. Die beiden Gelenkachsen Mᵤ und Mₒ sind durch ein Koppelglied 14 miteinander gelenkig verbunden. Konstruktiv wird diese Anordnung zweckmäßigerweise dadurch verwirklicht, daß sich die beiden Schienen 2a und 7a endseitig überlappen, und im Überlappungsbereich zwischen den Schienen 2a, 7a das Koppelglied 14 angeordnet ist und an einem Ende mit der Gelenkachse Mᵤ und am anderen Ende mit der Gelenkachse Mₒ gelenkig verbunden ist. Damit die überlappende Anordnung möglich ist, ist die Unterschenkelschiene 7a in medialer Richtung oder die Oberschenkelschiene 2a in lateraler Richtung gekröpft ausgebildet. Weiterhin ist zu erkennen, daß zweckmäßigerweise die Gelenkachse Mₒ der Oberschenkelschiene 2a des Medialgelenks 11 gegenüber der Gelenkachse Lₒ der Oberschenkelschiene 2 des Lateralgelenks 10 um das Versatzmaß Δx in Richtung auf das Oberschenkelteil versetzt ist. Weiterhin ist an der Seitenansicht gemäß Fig. 2 zu erkennen, daß zweckmäßigerweise die Gelenkachse Lₒ gegenüber der Gelenkachse Mₒ in Richtung posterior um das Versatzmaß Δy versetzt ist. Bei der gestreckten dimeren Kette des Lateralgelenks 10 bewegt sich das Rotationszentrum M₂ in Lᵤ relativ zu dem Rotationszentrum M₁ in Lₒ bzw. umgekehrt auf einer Kreisbahn um M₁ mit dem Radius RL, der dem Abstand der Achsen Lₒ und Lᵤ entspricht. RL ist dabei die relevante Länge des Koppelgliedes 13. Bei der überschlagenen dimeren Kette des Lateralgelenks 10 bewegt sich das Rotationszentrum M₃ in Mᵤ relativ zum Rotationszentrum M₄ in Mₒ bzw. umgekehrt auf einer Kreisbahn um das Rotationszentrum M₃ mit dem Radius R, der dem Abstand der Achsen Mᵤ und Mₒ entspricht, der wiederum die relevante Länge des Koppelgliedes 14 darstellt. Bei den vorstehenden Kreisbahnbewegungen treten jeweils auch Drehbewegungen um die jeweiligen Rotationszentren M₁ bis M₄ auf. In der Standstellung ist das Koppelglied 14 vertikal und parallel zum Koppelglied 13 angeordnet.

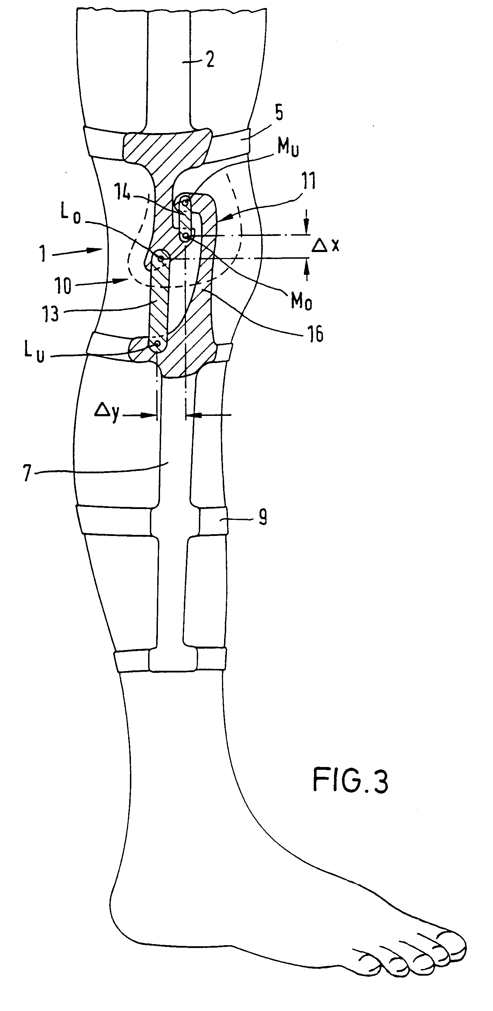

Während in den Fig. 1 und 2 dargestellt ist, wie die erfindungsgemäße Exoprothese ausgebildet ist, um das menschliche, kranke Knie beidseitig einzufassen, zeigt Fig. 3 eine Ausführungsform bei der die erfindungsgemäße Vier-Gelenkanordnung, einseitig am menschlichen, kranken Knie z.B. lateral, befestigt ist. Hierbei sind gleiche Teile wie in den Fig. 1 und 2 mit denselben Bezugsziffern versehen. Die Ausbildung des lateralen Gelenks 10 entspricht der Ausbildung gemäß den Fig. 1 und 2. Das mediale Gelenk 11 wird von einem Fortsatz 16 an der Unterschenkelschiene 7 gebildet, der das Lagerende der Oberschenkelschiene 2 überlappt und lateral gesehen in Richtung auf das menschliche Knie hinter dem Lagerende der Oberschenkelschiene 2 endet, so daß das Lateralgelenk 10 und das Medialgelenk 11 parallel zueinander angeordnet sind, wobei das Koppelglied 14 des Medialgelenks 11 ebenfalls in Richtung auf das Knie gesehen hinter der Oberschenkelschiene 2 über die Gelenkachse Mₒ an deren Lageransatz angelenkt ist.

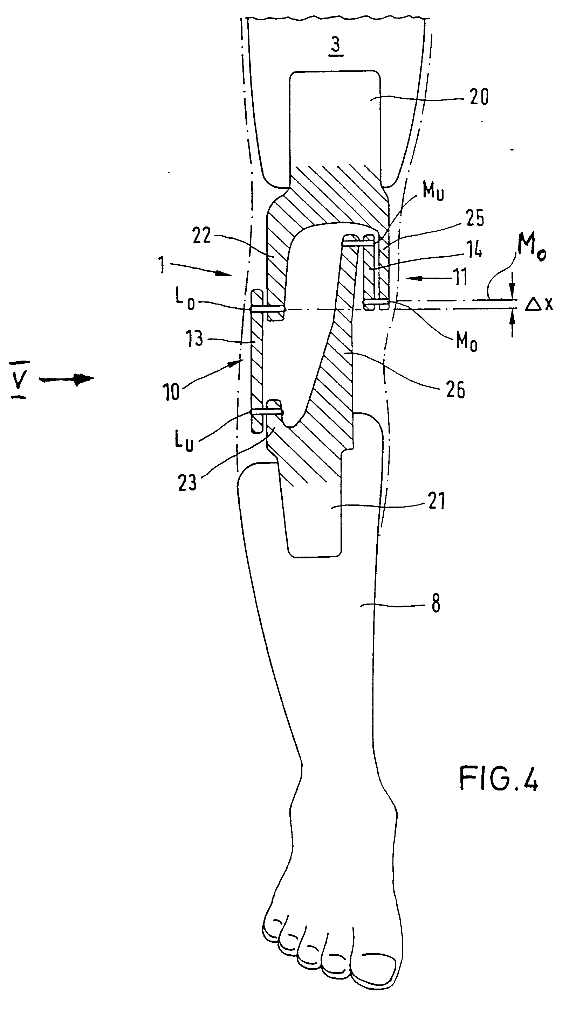

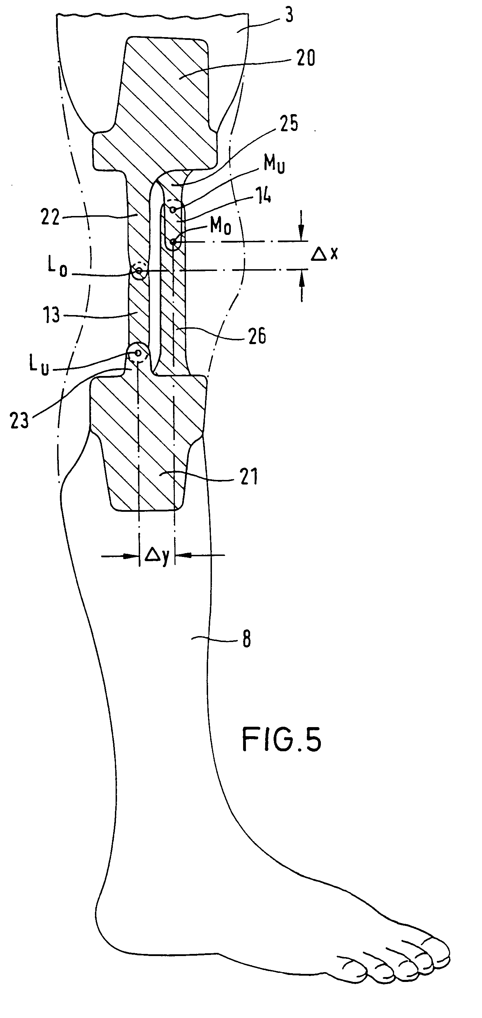

In den Fig. 4 und 5 ist eine Ausführungsform einer Exoprothese dargestellt, bei der durch diese das menschliche Kniegelenk vollständig dauerhaft ersetzt ist, wohingegen die Ausführungen gemäß den Fig. 1 bis 3 nur als Hilfseinrichtungen dienen sollen, so lange das vorhandene menschliche Knie selbst nicht in der Lage ist, die normale Gelenkfunktion durchzuführen. In Fig. 4 und 5 sind wiederum gleiche Teile wie in den Fig. 1 bis 3 mit denselben Bezugsziffern versehen. Das Lateralgelenk 10 und das Medialgelenk 11 sind hierbei an einem Oberschenkelstumpf 20 der implantierbar sein kann, und einem Unterschenkelstumpf 21, der Teil einer Unterschenkelprothese sein kann, befestigt. Das Lateralgelenk 10 wird von einem am Oberschenkelstumpf 20 ausgebildeten Lagerfortsatz 22 und einem am Unterschenkelstumpf 21 ausgebildeten Lagerfortsatz 23 gebildet, die über das Koppelglied 13 gelenkig verbunden sind, wozu das Koppelglied 13 über die Gelenkachse Lₒ am Lagerfortsatz 22 und über die Gelenkachse Lᵤ am Lagerfortsatz 23 gelenkig befestigt ist. Das Medialgelenk 11 wird von einem Gelenkfortsatz 25 am Oberschenkelstumpf 20 und einem Gelenkfortsatz 26 am Unterschenkelstumpf 21 gebildet, wobei der Gelenkfortsatz 26 den Gelenkfortsatz 25 überlappt und zwischen dem Gelenkfortsatz 25 und dem Lagerfortsatz 22 liegt. Das Koppelglied 14 ist zwischen den beiden Gelenkfortsätzen 25, 26 räumlich angeordnet und mit dem freien Ende des Gelenkfortsatzes 26 über die Gelenkachse Mᵤ gelenkig verbunden und mit dem freien Ende des Gelenkfortsatzes 25 über die Gelenkachse Mₒ. Wie sich aus dieser vorliegenden Darstellung ergibt, ist vorteilhafterweise ein Versatz des Lateralgelenks 10 nach posterior gegenüber dem Medialgelenk 11 um das Maß Δy vorgesehen und die Gelenkachse Mₒ ist gegenüber der Gelenkachse Lₒ um das Versatzmaß Δx in Richtung des Oberschenkels versetzt.

Weiterhin liegt es im Rahmen der Erfindung, wenn die Koppelglieder 13, 14 austauschbar befestigt sind, so daß unter Verwendung der übrigen Prothesenteile durch Veränderung der Länge der Koppelglieder 13, 14 die Gelenkgeometrie geändert und an die jeweiligen spezifischen Verhältnisse angepaßt werden kann. Auch kann erfindungsgemäß vorgesehen werden, daß die Länge der Oberschenkel- und Unterschenkelschienen variabel ausgebildet ist. Entsprechend können auch die Gelenkfortsätze gemäß Fig. 4 und 5 in ihrer Länge veränderbar ausgebildet sein. Zudem kann auch die Lagerstelle der Gelenkachsen Lₒ, Lᵤ bzw. Mᵤ, Mₒ so ausgebildet werden, daß sie in bezug auf ihre Höhe bzw. laterale Lagerstelle veränderbar angeordnet sind.

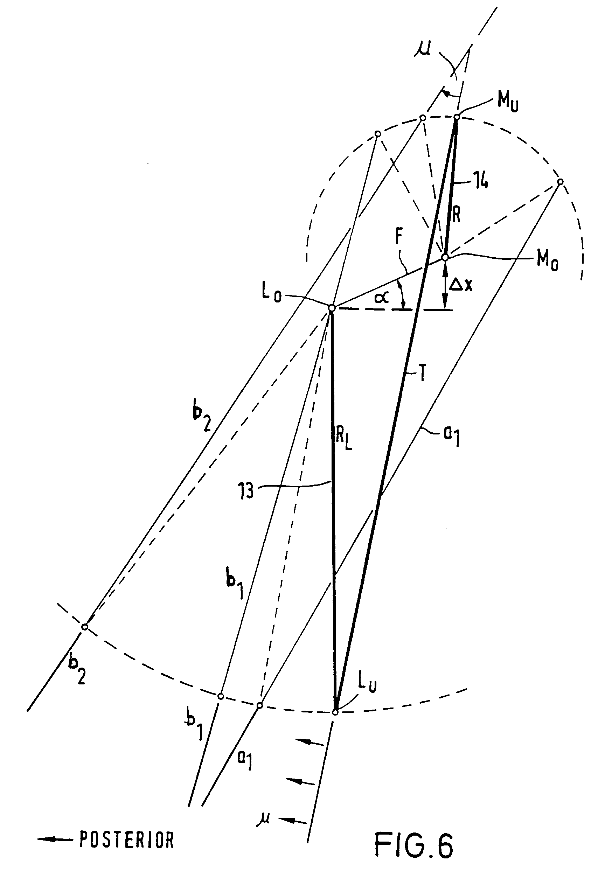

Fig. 6 zeigt die geometrischen Verhältnisse einer erfindungsgemäßen Exoprothese in seitlicher Ansicht gemäß Fig. 2. Die beiden Gelenke 10, 11 sind erfindungsgemäß so eingebaut, daß ihre Vier-Gelenkachsen Lₒ, Lᵤ und Mₒ, Mᵤ parallel zueinander in zwei parallelen Ebenen verlaufen, und daß das Lateralgelenk 10 gegenüber dem Medialgelenk 11 etwas nach posterior, d. h. nach hinten, um das Maß Δy versetzt ist. Ein derartiges Gelenkgebilde stellt ein Vier-Gelenk dar, wobei die mit F gekennzeichnete Gerade das Oberschenkelteil darstellt, und die mit T gekennzeichnete Gerade das Unterschenkelteil bildet. Bei der folgenden Betrachtung wird davon ausgegangen, daß das Oberschenkelteil F feststeht und das Gestell bildet. Die Relativbewegung des Unterschenkels gegenüber dem Oberschenkel stellt sich als die Bewegung des Teils T dar. Da die Länge von T größer ist als die Summe aus den Längen der Koppelglieder 13, 14, kann sich die Gelenkachse Lᵤ aus der dick gekennzeichneten Initialstellung heraus nur nach posterior bewegen. Die Gelenkachse Mᵤ kann sich sowohl nach anterior als auch nach posterior bewegen. In beiden Fällen schwenkt aber die distale Verlängerung von T, der Unterschenkel, nach hinten. Beide Fälle stellen zwei mögliche Kniebeugebewegungen dar, wobei jede einzelne für sich zwangläufig abläuft. Bei der anterioren Bewegung der Gelenkachse Mᵤ bewegt sich diese nach Überschreiten der anterioren Totlage (Koppelglied 14 und Gerade T bilden eine gerade Linie und fallen zusammen) weiter nach anterior. Das Unterschenkelteil kann dann die dünn gezeichnete Stellung a₁ einnehmen. Bei der posterioren Bewegung von Mᵤ erreicht diese Gelenkachse in der posterioren Totlage (Koppelglied 13 und Gerade T bilden eine gerade Linie und fallen zusammen, gezeichnete Lage b₁) ihre posteriorste Lage. Im Gefolge der weiteren Bewegung wandert Mᵤ dann in anteriore Richtung. Diese Wanderung geschieht so langsam, daß bei diesem weiteren Schwenken des Unterschenkelteils die Gelenkachse Mᵤ an ihrem Ort zu verharren scheint (Lage b₂ von T). In jedem Fall (a₁, b₁, b₂) ist der Unterschenkel nach hinten geschwenkt. Das künstliche Gelenk ist also so konstruiert, daß unter der Wirkung der kraftschlüssigen kompressiven Kräfte der Unterschenkel nur nach hinten schwenken kann.

Da das Medialgelenk 11 in Richtung auf den Oberschenkel hinsichtlich seiner Gelenkachsen Mᵤ und Mₒ um das Maß Δx versetzt ist, kann über die Größe dieses Versatzes der maximale Schwingungswinkel µ max. des Gelenks, der dem maximalen Beugewinkel des menschlichen Knies entspricht, nach posterior beeinflußt werden. Hierbei besteht die Gesetzmäßigkeit, daß je größer der Versatz Δx ist, um so kleiner der maximale Schwingungswinkel µ max. ausfällt. Vorzugsweise wird das Versatzmaß Δx derartig gewählt, daß die Verbindungsstrecke F von Lₒ nach Mᵤ gegenüber der Horizontallinie einen Winkel α einschließt, der zwischen 0 und 45° liegt: 0 < α < 45°. Auch liegt es im Rahmen der vorliegenden Erfindung, das Lateralgelenk 10 und das Medialgelenk 11 derart anzuordnen, daß die Ebenen durch ihre Koppelglieder 13, 14 räumlich zueinander geneigt verlaufen, woraus sich ein sphärischer Bewegungsablauf ergibt.

## Patentansprüche

1. Exoprothese für das menschliche Kniegelenk bestehend aus einem mit dem menschlichen Oberschenkel verbindbaren Oberschenkelteil (1) sowie eine mit dem menschlichen Unterschenkel verbindbaren oder diesen ersetzenden Unterschenkelteil (6), wobei das Unterschenkelteil (6) und das Oberschenkelteil (1) durch eine Vier-Gelenkanordnung verbunden ist, die aus zwei parallel zueinander angeordneten Teilgelenken, und zwar einem der Medialebene des menschlichen Knies zugewandten Medialgelenk (11) und einem zu diesem lateral nach außen angeordneten Lateralgelenk (10) besteht, die jeweils die Gelenkgeometrie einer Gelenkkette mit zwei Gelenkachsen (Mᵤ, Mₒ; Lₒ, Lᵤ) (dimere Gelenkkette) besitzen, wobei die Gelenkgeometrie des Medialgelenks (11) als eine überschlagene, dimere Gelenkkette und die Gelenkgeometrie des Lateralgelenks (10) als gestreckte dimere Kette ausgebildet ist, sowie die beiden Gelenkachsen des Lateralgelenks (10) und des Medialgelenks (11) jeweils mittels eines Koppelgliedes (13, 14) miteinander gelenkig verbunden sind,
wobei in der gestreckten Lage des Kniegelenks die Gelenkachse (Mᵤ) des Medialgelenks (11) zwischen dem Unterschenkelteil (6) und dem mit dem Oberschenkelteil (1) in der Gelenkachse (Mₒ) verbundenen Koppelgliedes (14) gegenüber der Gelenkachse (Mₒ) in Richtung auf das Oberschenkelteil (1) versetzt ist sowie die Gelenkachse (Lᵤ) des Lateralgelenks (10) des Unterschenkelteils (6), in der dieses mit dem Koppelglied (13) gelenkig verbunden ist, welches mit dem Oberschenkelteil (1) in der Gelenkachse (Lₒ) verbunden ist, gegenüber der Gelenkachse (Lₒ) in Richtung auf das Unterschenkelteil (6) versetzt ist,
**dadurch gekennzeichnet,** daß in der gestreckten Lage des Kniegelenks die Gelenkachsen (Lₒ, Lᵤ und Mᵤ, Mₒ) parallel zueinander in zwei parallelen Ebenen, die jeweils die Achsen des Medial- (Mᵤ, Mₒ) bzw. des Lateralgelenks (Lₒ, Lᵤ) enthalten, verlaufen, und das Lateralgelenk (10) gegenüber dem Medialgelenk (11) nach posterior (d.h. nach hinten) um ein Maß Δy, das dem Abstand der genannten parallelen Ebenen entspricht, sowie das Medialgelenk (11) gegenüber dem Lateralgelenk (10) hinsichtlich seiner Gelenkachsen Mᵤ und Mₒ in Richtung auf den Oberschenkel (3) um ein Maß Δx, das dem Abstand der Gelenkachsen Lₒ und Mₒ voneinander entspricht, versetzt ist.

2. Exoprothese nach Anspruch 1,
**dadurch gekennzeichnet,** daß das Oberschenkelteil (1) aus zwei Oberschenkelschienen (2, 2a) besteht, die an der inneren- und äußeren Seite eines Oberschenkels (3) eines menschlichen Beins (4) verlaufen und durch Querschienen (5) miteinander verbunden sind, die den Oberschenkel (3) umfassen und das Unterschenkelteil (6) ebenfalls aus zwei Unterschenkelschienen (7, 7a) gebildet ist, und zwar an der medialen- und lateralen Seite eines Unterschenkels (8) des menschlichen Beins (4), wobei die Schienen (7, 7a) durch Querschienen (9) verbunden sind, die den Unterschenkel (8) umfassen.

3. Exoprothese nach Anspruch 1 oder 2,
**dadurch gekennzeichnet,** daß die Zwei-Gelenke, das Lateralgelenk (10) und das Medialgelenk (11) an der lateralen Seite des menschlichen Knies gemeinsam angeordnet sind, wobei das mediale Gelenk (11) von einem Fortsatz (16) an der Unterschenkelschiene (7) gebildet ist, der das Lagerende der Oberschenkelschiene (2) in Richtung auf den Oberschenkel überlappt und lateral gesehen in Richtung auf das menschliche Knie hinter dem Lagerende der Oberschenkelschiene (2) endet, so daß das Lateralgelenk (10) und das Medialgelenk (11) parallel zueinander angeordnet sind, wobei das Koppelglied (14) des Medialgelenks (11) ebenfalls in Richtung auf das Knie gesehen hinter der Oberschenkelschiene über die Lagerachse Mᵤ an deren Lageransatz angelenkt ist.

4. Exoprothese nach Anspruch 1 oder 2,
**dadurch gekennzeichnet,** daß das Lateralgelenk (10) und das Medialgelenk (11) jeweils an einem Oberschenkelstumpf (20) und einem Unterschenkelstumpf (21), der ein Teil einer Unterschenkelprothese ist, befestigt sind, wobei das Lateralgelenk (10) von einem am Oberschenkelstumpf (20) ausgebildeten Lagerfortsatz (22) und einem an Unterschenkelstumpf (21) ausgebildeten Lagerfortsatz (23) gebildet wird, die über das Koppelglied (13) gelenkig verbunden sind, sowie das Medialgelenk (11) von einem Gelenkfortsatz (25) am Oberschenkelstumpf (20) und einem Gelenkfortsatz (26) am Unterschenkelstumpf (21) gebildet ist, wobei der Gelenkfortsatz (26) den Gelenkfortsatz (25) überlappt und zwischen dem Gelenkfortsatz (25) und dem Lagerfortsatz (22) liegt, sowie das Koppelglied (14) zwischen den beiden Gelenkfortsätzen (25, 26) angeordnet ist.

## Claims

1. Exoprosthesis for the human knee joint, consisting of a thigh section (1) which can be attached to the human thigh, and a lower leg section (6) which can be attached to the human lower leg or replace the latter, where the lower leg section (6) and the thigh section (1) are connected by a four-joint arrangement which consists of two parallel partial joints, namely a medial joint (11) facing the medial plane of the human knee and a lateral joint (10) arranged laterally to the outside thereof, each having the joint geometry of a joint chain with two articulation axes (Mᵤ, Mₒ; Lₒ, Lᵤ) (dimeric joint chain), where the joint geometry of the medial joint (11) is designed as a reversed dimeric joint chain and the joint geometry of the lateral joint (10) is designed as an extended dimeric chain, and the two articulation axes of the lateral joint (10) and of the medial joint (11) are in each case connected to one another in a hinged manner by means of a coupling member (13, 14), and where, in the extended position of the knee joint, the articulation axis (Mᵤ) of the medial joint (11), between the lower leg section (6) and the coupling member (14) connected to the thigh section (1) in the articulation axis (Mₒ), is offset in the direction towards the thigh section (1) with respect to the articulation axis (Mₒ), and the articulation axis (Lᵤ) of the lateral joint (10) of the lower leg section (6), in which the latter is connected in a hinged manner to the coupling member (13) which is connected to the thigh section (1) in the articulation axis (Lₒ), is offset in the direction towards the lower leg section (6) with respect to the articulation axis (Lₒ), characterized in that in the extended position of the knee joint, the articulation axes (Lₒ, Lᵤ and Mᵤ, Mₒ) run parallel to one another in two parallel planes which each contain the axes of the medial joint (Mᵤ, Mₒ) and of the lateral joint (Lₒ, Lᵤ), and the lateral joint (10) in relation to the medial joint (11) is offset posteriorly (i.e. to the rear) by a measurement Δy, which corresponds to the spacing of the said parallel planes, and the medial joint (11) in relation to the lateral joint (10) is offset, in respect of its articulation axes Mᵤ and Mₒ, in the direction towards the thigh (3) by a measurement Δx, which corresponds to the spacing of the articulation axes Lₒ and Mₒ from one another.

2. Exoprosthesis according to Claim 1, characterized in that the thigh section (1) consists of two thigh rails (2, 2a) which run along the inner side and outer side of a thigh (3) of a human leg (4) and are connected to one another by crossbars (5) which enclose the thigh (3), and the lower leg section (6) is likewise formed by two lower leg rails (7, 7a), on the medial side and lateral side of a lower leg (8) of the human leg (4), where the rails (7, 7a) are connected by crossbars (9) which enclose the lower leg (8).

3. Exoprosthesis according to Claim 1 or 2, characterized in that the two joints, the lateral joint (10) and the medial joint (11), are both arranged on the lateral side of the human knee, where the medial joint (11) is formed by an extension (16) of the lower leg rail (7) which overlaps the bearing end of the thigh rail (2) in the direction of the thigh and, viewed laterally in the direction of the human knee joint, terminates behind the bearing end of the thigh rail (2) so that the lateral joint (10) and the medial joint (11) are arranged parallel to one another, and the coupling member (14) of the medial joint (11), again viewed in the direction of the knee, is articulated behind the thigh rail via the bearing axis Mᵤ, on the bearing attachment thereof.

4. Exoprosthesis according to Claim 1 or 2, characterized in that the lateral joint (10) and the medial joint (11) are each secured on a thigh stump (20) and a lower leg stump (21), which forms a part of a lower leg prosthesis, where the lateral joint (10) is made up of a bearing extension (22) designed on the thigh stump (20) and of a bearing extension (23) designed on the lower leg stump (21), which extensions are connected to one another in a hinged manner via the coupling member (13), and the medial joint (11) is made up of a joint extension (25) on the thigh stump (20) and of a joint extension (26) on the lower leg stump (21), where the joint extension (26) overlaps the joint extension (25) and lies between the joint extension (25) and the bearing attachment (22), and the coupling member (14) is arranged between the two joint extensions (25, 26).

## Revendications

1. Exoprothèse pour l'articulation du genou humain, comprenant une partie (1) à relier à la cuisse humaine et une partie (6) à relier au bas de jambe humaine ou remplaçant celui-ci, la partie (6) et la partie (1) étant reliées entre elles par un agencement à quatre articulations consistant en deux articulations partielles disposées en parallèle l'une par rapport à l'autre, à savoir une articulation médiale (11) en regard du plan médial du genou humain, et une articulation latérale (10) associée latéralement vers l'extérieur à la précédente articulation, ces articulations présentant respectivement la géométrie articulaire d'une chaîne articulaire à deux axes (Mᵤ, Mₒ ; Lₒ, Lᵤ) (chaîne articulaire dimère), la géométrie articulaire de l'articulation médiale (11) ayant la forme d'une chaîne articulaire dimère repliée, et la géométrie de l'articulation latérale (10) ayant la forme d'une chaîne dimère allongée, les deux axes de l'articulation latérale (10) et de l'articulation médiale (11) étant respectivement articulés entre eux au moyen d'un élément d'accouplement (13, 14), l'axe d'articulation (Mᵤ) de l'articulation médiale (11) entre la partie du bas de jambe (6) et l'élément d'accouplement (14), relié à la partie de cuisse (1) par l'axe (Mₒ), étant décalé, dans la position allongée de l'articulation du genou, par rapport à l'axe d'articulation (Mₒ), dans la direction de la partie de cuisse (1), et l'axe (Lᵤ) de l'articulation latérale (10) de la partie du bas de jambe (6), par lequel cette articulation (10) est articulée à l'élément d'accouplement (13) relié à la partie de cuisse (1) par l'axe d'articulation (Lₒ), étant décalé par rapport à l'axe d'articulation (Lₒ) dans la direction de la partie du bas de jambe (6),
caractérisée en ce que, dans la position allongée du genou, les axes d'articulation (Lₒ, Lᵤ et Mᵤ, Mₒ) s'étendent parallèlement entre eux dans deux plans parallèles qui contiennent respectivement les axes de l'articulation médiale (Mᵤ, Mₒ) ou de l'articulation latérale (Lₒ, Lᵤ), et que l'articulation latérale (10) est décalée par rapport à l'articulation médiale (11) vers l'arrière d'une valeur Δy correspondant à la distance entre les plans parallèles susmentionnés, et les axes (Mᵤ, et Mₒ) de l'articulation médiale (11) sont décalés par rapport à l'articulation latérale (10) dans la direction de la cuisse (3) d'une valeur Δx correspondant à la distance entre les axes d'articulation (Lₒ) et (Mₒ).

2. Exoprothèse selon la revendication 1,
caractérisée en ce que la partie de cuisse (1) comprend deux barres (2, 2a) s'étendant le long de la face interne et externe de la cuisse (3) d'une jambe humaine (4) et reliées entre elles par des barres transversales (5) qui entourent la cuisse (3), et que la partie du bas de jambe (6) comprend également deux barres (7, 7a), à savoir sur la face médiale et latérale du bas de jambe (8) d'une jambe humaine (4), les barres (7, 7a) étant reliées entre elles par des barres transversales (9) qui entourent le bas de jambe (8).

3. Exoprothèse selon la revendication 1 ou 2,
caractérisée en ce que les articulations en deux parties, l'articulation latérale (10) et l'articulation médiale (11) sont agencées ensemble sur le côté latéral du genou humain, l'articulation médiale (11) étant formée par un prolongement (16) de la barre du bas de jambe (7) qui chevauche l'extrémité de l'appui de la barre de cuisse (2) dans la direction de la cuisse, et qui, vu latéralement dans la direction du genou humain, se termine derrière l'extrémité de l'appui de la barre de cuisse (2), de manière à ce que l'articulation latérale (10) et l'articulation médiale (11) soient agencées parallèlement l'une par rapport à à l'autre, et que l'élément d'accouplement (14) de cette articulation médiale (11) soit, vu également dans la direction du genou, articulé par l'axe (Mᵤ) derrière la barre de cuisse au prolongement d'appui de celle-ci.

4. Exoprothèse selon la revendication 1 ou 2,
caractérisée en ce que l'articulation latérale (10) et l'articulation médiale (11) sont respectivement fixées sur un moignon de cuisse (20) et un moignon de bas de jambe (21) qui fait partie d'une prothèse de bas de jambe, l'articulation latérale (10) étant formée par un prolongement d'appui (22) formé sur le moignon de cuisse (20), et par un prolongement d'appui (23) formé sur le moignon de bas de jambe (21), ces prolongements étant articulés entre eux par un élément d'accouplement (13), l'articulation médiale (11) étant à son tour formée par un prolongement d'articulation (25) sur le moignon de cuisse (20), et par un prolongement d'articulation (26) du moignon de bas de jambe (21), le prolongement d'articulation (26) chevauchant le prolongement d'articulation (25) et étant situé entre le prolongement d'articulation (25) et le prolongement d'appui (22), et l'élément d'accouplement (14) étant agencé entre les deux prolongements d'articulation (25, 26).
